(19) Europäisches Patentamt / European Patent Office / Office européen des brevets

(11) **EP 3 657 430 A1**

(12) **EUROPEAN PATENT APPLICATION**
published in accordance with Art. 153(4) EPC

(43) Date of publication:
**27.05.2020 Bulletin 2020/22**

(21) Application number: **18835835.2**

(22) Date of filing: **02.05.2018**

(51) Int Cl.:
*G06Q 50/22* (2018.01)    *A61B 5/00* (2006.01)

(86) International application number:
**PCT/JP2018/017506**

(87) International publication number:
**WO 2019/017039 (24.01.2019 Gazette 2019/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.07.2017 JP 2017139594**

(71) Applicant: **Sony Corporation**
**Tokyo 108-0075 (JP)**

(72) Inventors:
• **YANAGIMOTO, Takafumi**
**Tokyo 108-0075 (JP)**
• **IWAMURA, Atsushi**
**Tokyo 108-0075 (JP)**
• **HARA, Hiroshi**
**Tokyo 108-0075 (JP)**
• **OSHIMA, Koichi**
**Tokyo 108-0075 (JP)**

(74) Representative: **MFG Patentanwälte**
**Meyer-Wildhagen Meggle-Freund**
**Gerhard PartG mbB**
**Amalienstraße 62**
**80799 München (DE)**

(54) **INFORMATION PROCESSING DEVICE, INFORMATION PROCESSING METHOD, AND PROGRAM**

(57)    [Problem] A technique that can improve motivation of providers of biological information for healthcare is desired.

[Solution] An information processing device is provided that includes: a data acquisition unit configured to acquire information related to biological information, the biological information being provided by a provider; and a determination unit configured to determine a reward value provided to the provider as feedback for provision of the biological information, based on the information related to biological information.

FIG.1

EP 3 657 430 A1

**Description**

Field

The present disclosure relates to an information processing device, an information processing method, and a computer program.

Background

In recent years, techniques that determine the diathesis of providers based on biological information provided by the providers have been disclosed (for example, see Patent Literature 1).

Citation List

Patent Literature

Patent Literature 1: JP 2013-117941 A

Summary

Technical Problem

The biological information provided by the providers is often used as a reference to healthcare, but in most cases is not used for improving the providers' motivation for healthcare. It is therefore desired to provide a technique that can improve motivation of providers of biological information for healthcare.

Solution to Problem

According to the present disclosure, an information processing device is provided that includes: a data acquisition unit configured to acquire information related to biological information, the biological information being provided by a provider; and a determination unit configured to determine a reward value provided to the provider as feedback for provision of the biological information, based on the information related to biological information.
According to the present disclosure, an information processing method is provided that includes: acquiring information related to biological information, the biological information being provided by a provider; and determining a reward value provided to the provider as feedback for provision of the biological information, based on the information related to biological information.
According to the present disclosure, a computer program is provided that causes a computer to function as an information processing device that includes: a data acquisition unit configured to acquire information related to biological information, the biological information being provided by a provider; and a determination unit configured to determine a reward value provided to the provider as feedback for provision of the biological information, based on the information related to biological information.

Advantageous Effects of Invention

As explained above, the present disclosure provides a technique that can improve motivation of providers of biological information for healthcare. The effect above is not always limitative, and any effects illustrated in the present description or other effects that can be understood from the present description may be achieved in addition to the effect above or instead of the effect above.

Brief Description of Drawings

FIG. 1 is a diagram illustrating a configuration example of an information processing system according to a first embodiment of the present disclosure.
FIG. 2 is a block diagram illustrating a functional configuration example of a data management system according to the first embodiment.
FIG. 3 is a diagram for explaining the overall functions of the information processing system according to the first embodiment.

FIG. 4 is a diagram illustrating an example of an evaluation function.

FIG. 5 is a diagram illustrating an example of the tendency of change in vital data.

FIG. 6 is a diagram illustrating a configuration example of an information processing system according to a second embodiment of the present disclosure.

FIG. 7 is a diagram for explaining the overall functions of the information processing system according to the second embodiment of the present disclosure.

FIG. 8 is a diagram illustrating an example of a search screen displayed by a research institution terminal.

FIG. 9 is a diagram illustrating an example of a search result screen displayed by the research institution terminal.

FIG. 10 is a diagram illustrating an example of a detail data screen displayed by the research institution terminal.

FIG. 11 is a diagram illustrating a configuration example of an information processing system according to a third embodiment of the present disclosure.

FIG. 12 is a diagram for explaining the overall functions of the information processing system according to the third embodiment of the present disclosure.

FIG. 13 is a diagram illustrating an example of a search result screen displayed by a selling institution terminal.

FIG. 14 is a diagram illustrating an example of an advertisement screen displayed by a provider terminal.

FIG. 15 is a block diagram illustrating a hardware configuration example of the data management system according to the embodiments of the present disclosure.

Description of Embodiments

Preferred embodiments of the present disclosure will be described in detail below with reference to the accompanying drawings. In the present description and drawings, components having substantially the same functional configuration are denoted by the same reference signs and an overlapping description is omitted.

In the present description and drawings, a plurality of components having substantially the same or similar functional configuration may be denoted by the same reference signs followed by different numerals to be distinguished from each other. However, when the components having substantially the same or similar functional configuration need not be distinguished from each other, they are denoted only by the same reference signs. Similar components in different embodiments may be denoted by the same reference signs followed by different alphabets to be distinguished from each other. However, when the similar components need not be distinguished from each other, they are denoted only by the same reference signs.

The description will be given in the following order.

0. Overview
1. First Embodiment

    1.1. System Configuration Example
    1.2. Functional Configuration Example of Data Management System
    1.3. Detail of Functions of Information Processing System

2. Second Embodiment

    2.1. System Configuration Example
    2.2. Detail of Functions of Information Processing System

3. Third Embodiment

    3.1. System Configuration Example
    3.2. Detail of Functions of Information Processing System

4. Hardware Configuration Example
5. Closing

<0. Overview>

Techniques that determine the diathesis of providers based on biological information provided by the providers have been disclosed. The biological information provided by the providers is often used as a reference to healthcare, but in most cases is not used for improving the providers' motivation for healthcare. In the present embodiment, a technique that can improve motivation of providers of biological information for healthcare will mainly be described.

The overview of an embodiment of the present disclosure has been described above.

<1. First Embodiment>

First of all, a first embodiment of the present disclosure will be described.

[1.1. System Configuration Example]

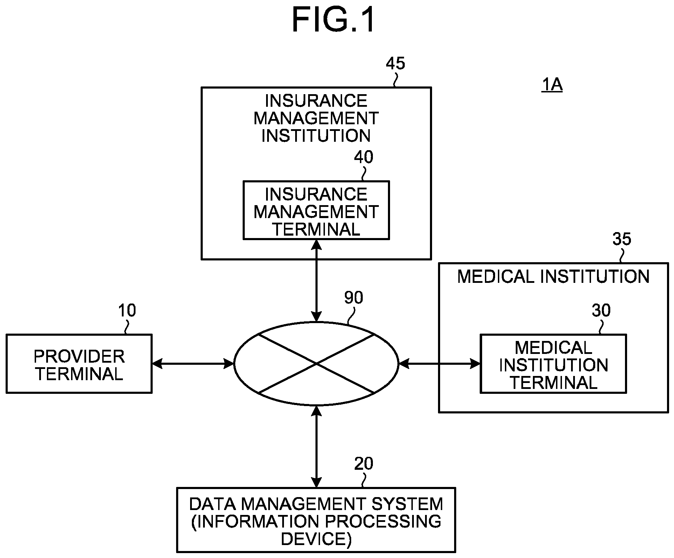

A configuration example of an information processing system 1A according to the first embodiment of the present disclosure will now be described. FIG. 1 is a diagram illustrating a configuration example of the information processing system 1A according to the first embodiment of the present disclosure. As illustrated in FIG. 1, the information processing system 1A according to the first embodiment of the present disclosure has a provider terminal 10, an information processing device (which hereinafter may be referred to as "data management system") 20, a medical institution terminal 30, and an insurance management terminal 40.

The insurance management terminal 40 is used in an insurance management institution 45. An example of the insurance management institution 45 is a local government or the Ministry of Health, Labour and Welfare. The medical institution terminal 30 is used in a medical institution 35. The provider terminal 10, the data management system (information processing device) 20, the medical institution terminal 30, and the insurance management terminal 40 are connected to a network 90 and configured to be able to communicate through the network 90.

In the following description, it is mainly assumed that a variety of data is transmitted/received between devices connected to the network 90. However, instead of data transmission/reception between the devices through the network 90, data may be read from the devices as appropriate, the data may be exchanged from people to people by hand delivery of a predetermined medium or by mail of a predetermined medium, and the data may be input to the devices as appropriate. The predetermined medium is not limited to a specific medium. For example, the predetermined medium may be a paper medium or a computer-readable recording medium.

A configuration example of the information processing system 1A according to the first embodiment of the present disclosure has been described above.

[1.2. Functional Configuration Example of Data Management System]

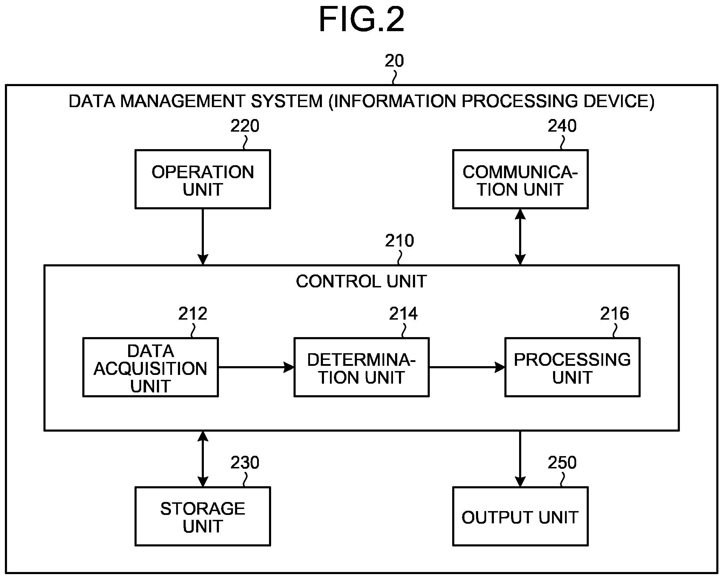

A functional configuration example of the data management system 20 according to the first embodiment of the present disclosure will now be described. FIG. 2 is a block diagram illustrating a functional configuration example of the data management system 20 according to the first embodiment of the present disclosure. As illustrated in FIG. 2, the data management system 20 includes a control unit 210, an operation unit 220, a storage unit 230, a communication unit 240, and an output unit 250. These functional blocks of the data management system 20 will be described below.

The control unit 210 may be configured with, for example, one or more processing devices such as central processing units (CPUs). When the blocks are configured with a processing device such as a CPU, such a processing device may be configured with an electronic circuit. The control unit 210 includes a data acquisition unit 212, a determination unit 214, and a processing unit 216. These functional blocks will be described in detail later.

The operation unit 220 has the function of accepting input of operation by an organizer. In the present embodiment, a case where the operation unit 220 is configured with a touch panel is mainly described. The operation unit 220, however, is not limited to a touch panel. For example, the operation unit 220 may include a mouse, a keyboard, a switch, and a lever. The operation unit 220 may include a microphone detecting voice of the organizer.

The storage unit 230 includes a memory and is a recording device that stores a computer program executed by the control unit 210 and stores data necessary for execution of the computer program. The storage unit 230 temporarily stores data for arithmetic operation by the control unit 210. The storage unit 230 may be a magnetic storage device, may be a semiconductor storage device, may be an optical storage device, or may be a magnetic optical storage device.

The communication unit 240 includes a communication circuit and has the function of communicating with another device through a network. For example, the communication unit 240 is configured with a communication interface. For example, the communication unit 240 can communicate with the provider terminal 10, with the medical institution terminal 30, and with the insurance management terminal 40 through the network 90.

The output unit 250 outputs a variety of information. For example, the output unit 250 may include a display capable of providing display visible to the organizer. The display may be a liquid crystal display or may be an organic electro-luminescence (EL) display. The output unit 250 may include a sound output device such as a speaker. Alternatively, the output unit 250 may include a tactile sense presenting device that presents tactile sense to the organizer.

A functional configuration example of the data management system 20 according to the first embodiment of the present disclosure has been described above.

[1.3. Detail of Functions of Information Processing System]

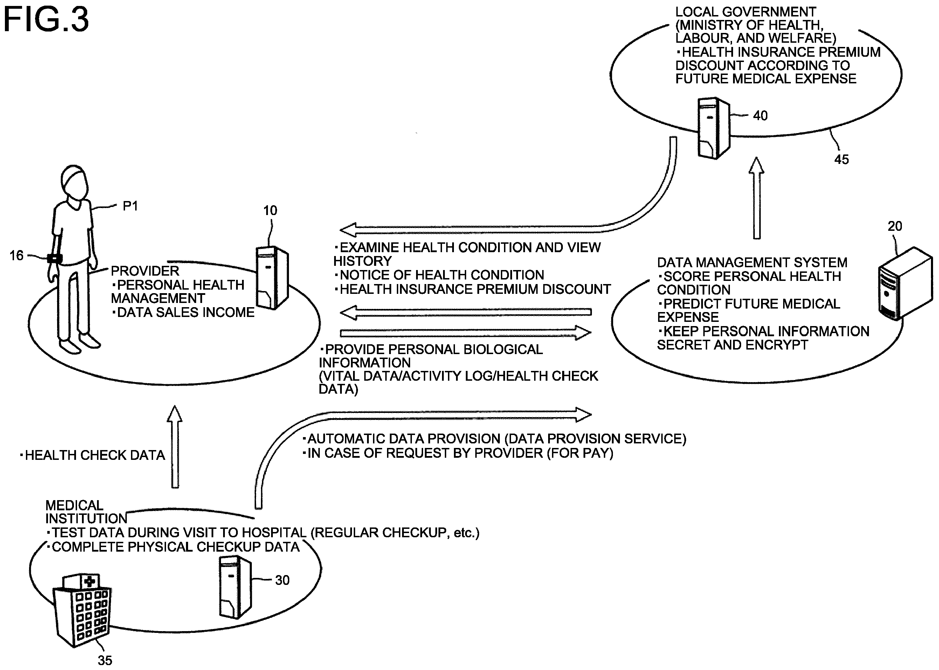

The detail of functions of the information processing system 1A according to the first embodiment of the present disclosure will now be described. FIG. 3 is a diagram for explaining the overall functions of the information processing system 1A according to the first embodiment of the present disclosure. As illustrated in FIG. 3, in the first embodiment of the present disclosure, in addition to a provider P1 and the provider terminal 10, the data management system 20, the medical institution terminal 30 used in the medical institution 35, and the insurance management terminal 40 used in the insurance management institution 45 are present.

In the medical institution 35, health check data of the provider P1 is obtained. Specifically, the health check data of the provider P1 may include test data obtained through a test (for example, test in a regular checkup) on the provider P1 during visit to a hospital or may include data of a complete physical checkup conducted on the provider P1. The health check data of the provider P1 obtained in the medical institution 35 is presented to the provider P1. The health check data may correspond to an example of biological information. The health check data may include data difficult to measure personally and may serve as effective data.

The health check data of the provider P1 is transmitted from the medical institution terminal 30 to the provider terminal 10, received by the provider terminal 10, and displayed by the provider terminal 10 to be presented to the provider P1.

The provider P1 wears a wearable device 16 containing a sensor, and the sensor detects sensor data (vital data). Here, the sensor detecting vital data is not limited to a specific type. For example, the sensor detecting vital data may be embedded in a medical instrument for home use or may be installed indoors or outdoors. The vital data may correspond to an example of biological information. While health check data is obtained in a particular condition such as on an empty stomach, vital data is also used as an example of the biological information, whereby biological information obtained in various conditions is used.

The vital data is not limited to a specific kind. For example, the vital data may include at least one of heart rate, blood pressure, blood sugar level, breathing rate, body temperature, brain wave, electrocardiogram, blood flow, cardiac sound, blood oxygen level, cholesterol, blood-alcohol level, perspiration, myogram, and the amount of physical activity of the provider P1. The amount of physical activity may include history (log) of the amount of physical activity. The amount of physical activity may be any amount related to physical activity of the provider P1. For example, the amount of physical activity may include the number of steps, may include walking distance, or may include calories burned.

The vital data is transmitted from the provider terminal 10 to the data management system 20 and received by the data management system 20. The health check data is transmitted from the provider terminal 10 to the data management system 20 and received by the data management system 20. Alternatively, the health check data may be directly transmitted from the medical institution terminal 30 to the data management system 20 and received by the data management system 20. For example, such direct transmission of health check data may be performed for pay, for example, when the provider P1 subscribes a data provision service by a medical institution.

The biological information (for example, vital data, health check data) provided by the provider P1 is received by the communication unit 240 in the data management system 20. In another case, when attribute data (or setting data) of the provider P1 is transmitted from the provider terminal 10, the attribute data (or setting data) is received by the communication unit 240 in the data management system 20. The biological information and the attribute data (or setting data) received by the communication unit 240 may be private information and therefore kept secret, for example, through encryption and then securely stored in the storage unit 230.

Examples of the attribute data (or setting data) of the provider P1 include name, domicile, age, gender, disease information, drug usage information, clinical research consent, and whether to deliver healthcare information. The attribute data (or setting data) of the provider P1 can be used for utilization of biological information in the data management system 20. The data acquisition unit 212 acquires information related to this biological information.

The determination unit 214 determines a reward value provided to the provider P1 as feedback for provision of biological information, based on the information related to biological information. This configuration can improve the motivation of the provider P1 of biological information for healthcare. This configuration also encourages the provider P1 to provide biological information, and it is expected that biological information by provider P1 is utilized more efficiently. The processing unit 216 performs a predetermined process according to the reward value.

In the first embodiment of the present disclosure, it is mainly assumed that the reward value is the amount of money. However, the reward value is not limited to the amount of money. The reward value may be a point having a value equivalent to the amount of money. Specifically, in the first embodiment of the present disclosure, it is mainly assumed that the reward value is a discount on a health insurance premium. However, the reward value may be a discount on an insurance premium other than health insurance premiums.

In the first embodiment of the present disclosure, it is mainly assumed that the information related to biological information includes a calculation result (hereinafter also referred to as "overall score") calculated based on biological information. However, as explained in other embodiments, the information related to biological information is not limited to the overall score. In the first embodiment of the present disclosure, it is mainly assumed that the calculation of the overall score is performed by the data acquisition unit 212. However, the calculation of the overall score may be performed outside the data management system 20.

The calculation of the overall score (scoring of biological information) is specifically described. However, the calculation of the overall score may be performed by any method. For example, the data acquisition unit 212 calculates the overall score, based on at least one of an evaluation value of biological information, a period in which biological information is kept in a predetermined range, the tendency of change in biological information, the amount of physical activity of the provider when biological information is detected, and the confidence of biological information. An example in which the data acquisition unit 212 calculates the overall score based on all of them will be described below by way of example.

The evaluation value of the biological information may be the difference between the value indicated by biological information provided by the provider P1 and a standard value. The smaller the difference, the larger a first score (health score). The period in which biological information is kept in a predetermined range is a period in which the value indicated by biological information continuously falls between a predetermined upper limit value and a predetermined lower limit value. The longer the period, the larger a second score (maintenance score).

The tendency of change in biological information may be whether the tendency of change in biological information is "improving tendency" or "deteriorating tendency" or may be any other tendency. When the tendency of change in biological information is "improving tendency", a third score (improvement score) is larger than when the tendency of change in biological information is "deteriorating tendency". The amount of physical activity of the provider when biological information is detected may be the amount of physical activity or history of the amount of physical activity. The larger the amount of physical activity, the larger a fourth score (the amount of physical activity score). The larger the confidence of biological information, the larger a fifth score.

The overall score can be calculated based on these first score (health score), second score (maintenance score), third score (improvement score), fourth score (the amount of physical activity score), and fifth score (confidence). For example, the overall score may be calculated by adding up these scores. When only some of these scores are used, the overall score may be calculated by adding up some scores.

The data acquisition unit 212 may calculate the confidence of biological information, based on at least one of the number of samples of biological information, the sampling period of biological information, and the average sampling interval of biological information. An example in which the data acquisition unit 212 calculates the confidence of biological information based on all of them will be described below by way of example.

An example of scoring will be described below, taking vital data (for example, blood sugar level) obtained from a 40-year-old male using the wearable device 16 as an example. In this case, the wearable device 16 can measure a blood sugar level and the amount of physical activity and has the function of storing biological information into a storage device contained in the wearable device 16 or an external storage device (for example, a cloud storage).

When the number of samples is n (for example, n days when the sampling interval is one day), each individual sampling interval is $t_{n-m} - t_{n-m-1}$, and the sampling period p in total is $p = t_n - t_1$. Based on these, the average sampling interval is calculated: $\Delta t_{av} = \sum(t_{n-m} - t_{n-m-1})/n$.

In this case, it can be determined that the larger the number of samples n, the longer the sampling period p, and the closer the average sampling interval $\Delta t_{av}$ to regular intervals, the higher the confidence of vital data. Then, when the function for calculating the confidence of vital data is t(), the confidence T is written as $T = t(n, p, \Delta t_{av})$.

The data acquisition unit 212 also can classify the vital data (for example, blood sugar level) according to a state of the provider P1. For example, the data acquisition unit 212 can classify the vital data (for example, blood sugar level) into any one of fasting data $a_{n-m}$, after-meal data $b_{n-m}$, and post-exercise data $c_{n-m}$, based on a parameter in detecting vital data. The parameter may include at least one of the amount of physical activity, action history, positional information, and the amount of change in blood sugar level of the provider P1.

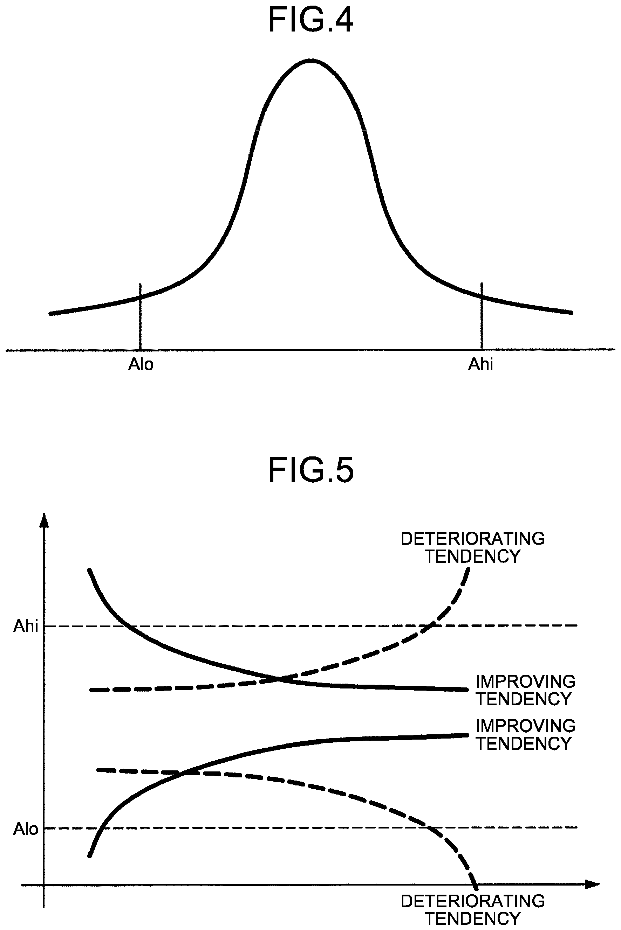

Here, an example of the method of evaluating fasting data $a_{n-m}$ is described by way of example. FIG. 4 is a diagram illustrating an evaluation function. The blood sugar level on an empty stomach is indexed by a value of 70 to 99 (mg/dl). When the upper limit value of the blood sugar level on an empty stomach is $A_{hi}$ and the lower limit value is $A_{lo}$, the evaluation value on an empty stomach $S_{n-m}$ can be calculated by an evaluation function $S_{n-m}(A_{hi}, A_{lo}, a_{n-m})$ as illustrated in FIG. 4.

Then, the function for evaluating the evaluation value $S_{n-m}$ in chronological order is set as $F_A(S_n, S_{n-1}, ..., S_1)$. FIG. 5 is a diagram illustrating an example of the tendency of change in vital data. As illustrated in FIG. 5, when the evaluation value tends to fall between the upper limit value $A_{hi}$ and the lower limit value $A_{lo}$, it can be said that the tendency of change in evaluation value is "improving tendency". On the other hand, as illustrated in FIG. 5, when the evaluation value tends to fall outside between the upper limit value $A_{hi}$ and the lower limit value $A_{lo}$, it can be said that the tendency of change in evaluation value is "deteriorating tendency". When the evaluation value is "improving tendency", the improvement score is higher than when the evaluation value is "deteriorating tendency".

Then, the overall score $A_{score}$ can be calculated by the function $A_{score}(S, F_A, T)$ using the evaluation value S of vital data, the tendency $F_A$ of change in vital data, and the confidence T of vital data. Although in the example described here, the overall score is calculated using the evaluation value of vital data, the tendency of change in vital data, and the confidence of vital data, the overall score may be calculated based on the amount of physical activity ($act_n$, $act_{n-1}$..., $act_1$) in addition to these data.

In the foregoing description, the case where the biological information constitutes one item (in the example above, blood sugar level) has mainly been described. However, the biological information may constitute a plurality of items (for example, blood sugar level and blood pressure). In such a case, the data acquisition unit 212 may calculate a total score based on the overall score for each item and the weight for each item, and the total score may be used instead of the overall score.

The data acquisition unit 212 may calculate the weight of an item, based on at least one of the amount of physical activity of the provider P1 and the confidence of biological information. However, the weight may be calculated by any method. For example, the weight may be calculated such that it is larger for an item with a larger amount of physical activity in the sampling period (provision period) of biological information. Alternatively, the weight may be calculated such that it is larger for an item with a larger confidence.

The calculation of the overall score has been described. Subsequently, the determination unit 214 determines a discount on a health insurance premium, based on the overall score. For example, since it can be assumed that the higher the overall score, the lower the risk for suffering a disease, the discount can be determined to be high. In this way, discounting a health insurance premium can reduce the burden of healthcare cost on the provider P1. The processing unit 216 calculates the health insurance premium to be paid by the provider P1 by deducting the discount from the health insurance premium as a basis. An example of the relation between the overall score and the discount on a health insurance premium will now be described.

An example of the relation between the overall score and the discount on a health insurance premium will be described below, taking a 40-year-old male living in Setagaya-ku, Tokyo with an annular income of 4 million yen and a net income of 2.66 million yen (standard amount = 2.33 million yen) as an example. The national health insurance premium is calculated as the sum of an income-based levy and a per capita levy. As an example, the breakouts of the income-based levy and the per capita levy in 2016 are as follows.

```
       Income-based levy (basic portion + elderly support portion
  + nursing care insurance portion)
       basic portion = standard amount × 6.86%


          elderly support portion = standard amount × 2.02%


          nursing care insurance portion = standard amount × 1.52%
          Per capita levy (basic portion + elderly support portion +
     nursing care insurance portion)


       basic portion= the number of insurance members in a household ×
  35,400 yen

          elderly support portion = the number of insurance members in a
  household × 10,800 yen


          nursing care insurance portion = the number of insurance members
  in a household × 14,700 yen
```

Here, it is possible to predict the individual risk of suffering a disease in the future from the individual overall score described above (because the overall score can be derived, for example, from the individual health condition, the tendency of change in individual vital data, and the confidence of vital data). For example, for an individual whose risk of suffering a disease is lower than a threshold, a possible discount method is discounting the ratio (here, 6.86%) of the income-based levy of the basic portion allocated to the medical expense born by a local government or deducting the personal portion of the per capita levy ($1 \times 35,400$ yen).

In a case of an individual over 40 years old, a similar discount method may be applied to the nursing care insurance portion of the income-based levy and the per capita levy.

Returning to FIG. 3, we continue the description. The health insurance premium to be paid by the provider P1 is transmitted by the communication unit 240 to the insurance management terminal 40 and displayed by the insurance management terminal 40. The insurance management institution 45 collects the discounted health insurance premium from the provider P1. The health insurance premium to be paid by the provider P1 is also transmitted by the communication unit 240 to the provider terminal 10 and displayed by the provider terminal 10.

In addition, the health condition (for example, overall score) calculated by the data acquisition unit 212 is transmitted by the communication unit 240 to the provider terminal 10 and displayed by the provider terminal 10. This process enables the provider P1 to grasp his/her health condition. When the provider P1 inputs a request to the provider terminal 10 and the request is transmitted to the data management system 20, the health condition calculated in the past is transmitted by the communication unit 240 to the provider terminal 10 and displayed by the provider terminal 10. This process enables the provider P1 to view the history of his/her health condition.

The first embodiment of the present disclosure has been described above.

<2. Second Embodiment>

A second embodiment of the present disclosure will now be described.

[2.1. System Configuration Example]

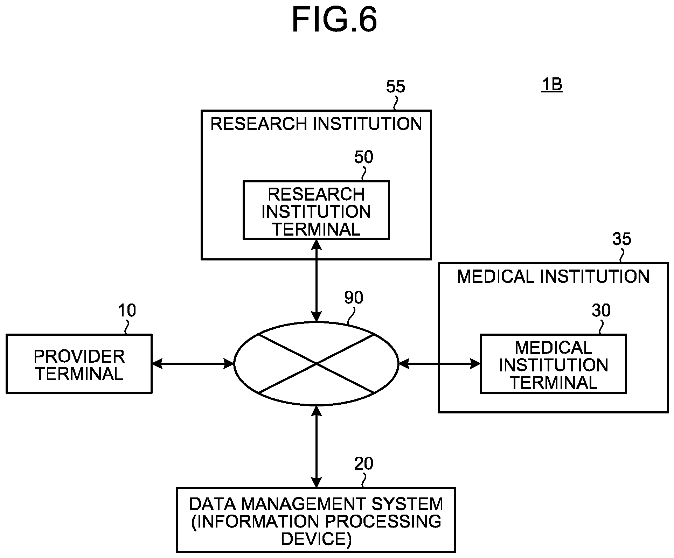

A configuration example of an information processing system 1B according to the second embodiment of the present disclosure will now be described. FIG. 6 is a diagram illustrating a configuration example of the information processing system 1B according to the second embodiment of the present disclosure. As illustrated in FIG. 6, the information processing system 1B according to the second embodiment of the present disclosure differs from the information processing system 1A according to the first embodiment of the present disclosure in that it has a research institution terminal 50 instead of the insurance management terminal 40.

The research institution terminal 50 is used in a research institution 55. Examples of the research institution 55 include hospitals, universities, and pharmaceutical companies. In the configuration of the information processing system 1B according to the second embodiment of the present disclosure, the same configuration as the configuration of the information processing system 1A according to the first embodiment of the present disclosure will not be further elaborated, and a different configuration is mainly described below.

A configuration example of the information processing system 1B according to the second embodiment of the present disclosure has been described above.

[2.2. Detail of Functions of Information Processing System]

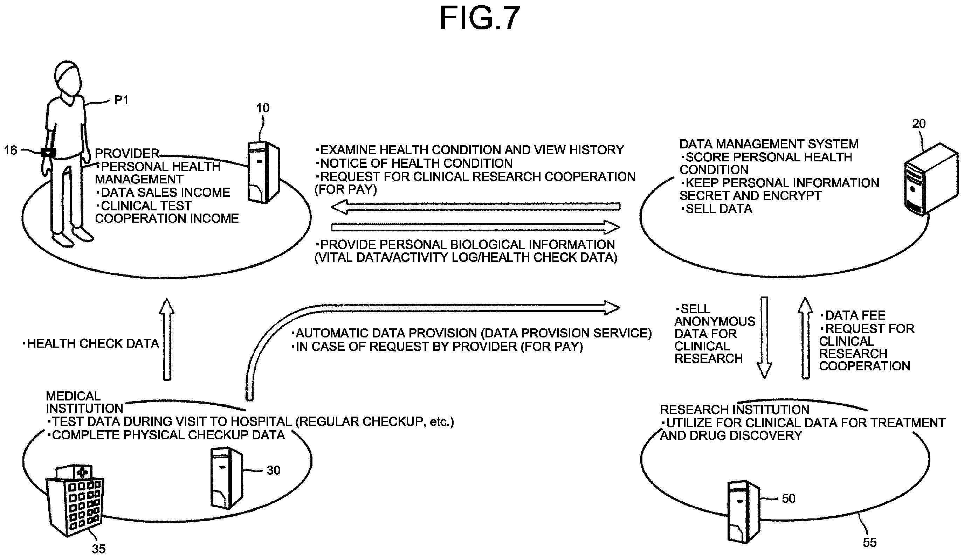

The detail of functions of the information processing system 1B according to the second embodiment of the present disclosure will now be described. FIG. 7 is a diagram for explaining the overall functions of the information processing system 1B according to the second embodiment of the present disclosure. As illustrated in FIG. 7, the second embodiment of the present disclosure differs from the first embodiment of the present disclosure in that the research institution terminal 50 used in the research institution 55 is present instead of the insurance management terminal 40 used in the insurance management institution 45.

As described in the first embodiment of the present disclosure, the data acquisition unit 212 acquires information related to biological information. The determination unit 214 determines a reward value provided to the provider P1 as feedback for provision of biological information, based on information related to biological information. The processing unit 216 performs a predetermined process according to the reward value.

Also in the second embodiment of the present disclosure, it is mainly assumed that the reward value is the amount of money in the same manner as in the first embodiment of the present disclosure. Specifically, in the second embodiment of the present disclosure, it is assumed that the research institution 55 asks for cooperation in clinical research (for example, as illustrated in FIG. 7, through the data management system 20), and biological information is provided from the provider P1 as biological information to be used in clinical research in the research institution 55.

The provider P1 provides biological information as the biological information used in clinical research, whereby the research institution 55 can search for desired biological information (for example, for pay) in chronological order in addition to age, gender, established disease, drug usage and can obtain subject data useful for clinical research. In such a case, in the second embodiment of the present disclosure, it is mainly assumed that the reward value is the amount of money (research cooperation fee) paid to the provider P1 for cooperation on clinical research.

The information related to biological information may include the data size of biological information provided by the provider P1 (may be pay-per-use), may include the number of items that constitute the biological information provided by the provider P1, or may include the period in which the biological information provided by the provider P1 is provided (for example, per month when a monthly use agreement is made, or per year when an annual use agreement is made). The determination unit 214 determines the research cooperation fee to be paid to the provider P1, based on the information related to biological information.

In this way, the research cooperation fee is paid to the provider P1 to encourage the provider P1 to provide biological information. The biological information by the provider P1 is thus used in research more efficiently, which increases the possibility that undiscovered treatment for a disease is found early. In addition, the time taken for research development of disease treatment and drug discovery can be reduced, and it is expected that research spending can be reduced.

The processing unit 216 may make a settlement process of transferring the research cooperation fee to the account of the provider P1. The research cooperation fee is transmitted by the communication unit 240 to the research institution terminal 50 and displayed by the research institution terminal 50. The research cooperation fee is also transmitted by the communication unit 240 to the provider terminal 10 and displayed by the provider terminal 10.

For example, it is thought that the larger the data size of biological information provided by the provider P1 is, the larger the contribution by the provider P1 to clinical research is. Accordingly, the larger the data size of biological information provided by the provider P1 is, the higher research cooperation fee provided to the provider P1 may be set by the determination unit 214. If so, it is expected that the provider P1 is motivated to provide biological information of a larger data size.

It is thought that the larger the number of items of biological information provided by the provider P1 is, the larger the contribution by the provider P1 to clinical research is. Accordingly, the larger the number of items that constitute the biological information provided by the provider P1 is, the higher research cooperation fee paid to the provider P1 may be set by the determination unit 214. If so, it is expected that the provider P1 is motivated to provide biological information including more items.

It is thought that the longer the period in which biological information is provided by the provider P1 is, the larger the contribution by the provider P1 to clinical research is. Accordingly, the longer the period in which biological information is provided by the provider P1 is, the higher research cooperation fee provided to the provider P1 may be set by the determination unit 214. If so, it is expected that the provider P1 is motivated to provide biological information for a longer period.

The biological information provided by the provider P1 is used (for example, as anonymous data) in whole or in part for clinical research in the research institution 55. In this way, when there is a user (research institution 55) of the biological information provided by the provider P1 in whole or in part, a data usage fee may be provided from the research institution 55 to the data management system 20. In this case, the determination unit 214 determines the amount of money (data usage fee) to be paid by the research institution 55, based on information related to biological information that is used by the user in the biological information provided by the provider P1.

The information related to biological information used by the research institution 55 (user) may include the data size of biological information used by the research institution 55 (user) (may be pay-per-use), or may include the number of items that constitute the biological information used by the research institution 55 (user), or may include the period in which biological information is used by the research institution 55 (user) (for example, per month when a monthly use agreement is made, or per year when an annual use agreement is made). The determination unit 214 determines a data usage fee, based on the information related to the biological information used by the research institution 55 (user). The processing unit 216 may perform a settlement process of withdrawing the data usage fee from the account of the research institution 55 (user).

The data usage fee is transmitted by the communication unit 240 to the research institution terminal 50 and displayed by the research institution terminal 50. The data usage fee is transmitted by the communication unit 240 to the provider terminal 10 and displayed by the provider terminal 10.

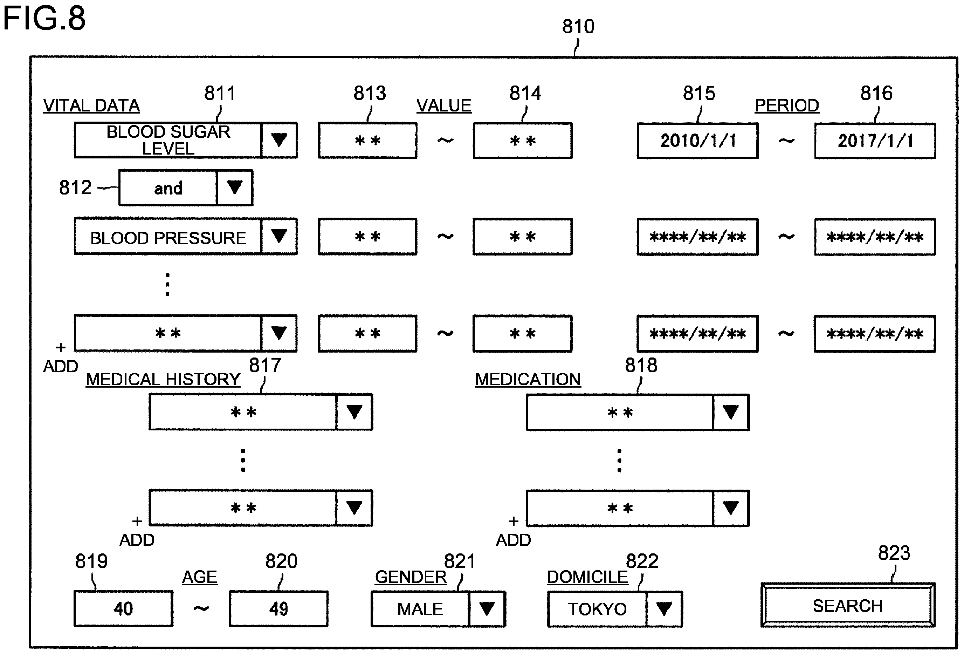

The data usage in the research institution 55 will be described. FIG. 8 is a diagram illustrating an example of a search screen displayed by the research institution terminal 50. As illustrated in FIG. 8, a search screen 810 displayed by the research institution terminal 50 includes a vital data item select field 811, a vital data upper limit value input field 813, a vital data lower limit value input field 814, a start date of vital data provision period input field 815, and an end date of vital data provision period input field 816. When a plurality of items are searched, an item-to-item relation input field 812 is provided.

The search screen 810 additionally includes a medical history of provider P1 select field 817, a medication of provider P1 select field 818, an age lower limit value input field 819, an age upper limit value input field 820, a gender select field 821, and a domicile select field 822. When a search button 823 is pressed, vital data that satisfies these conditions is retrieved from vital data provided from one or more providers.

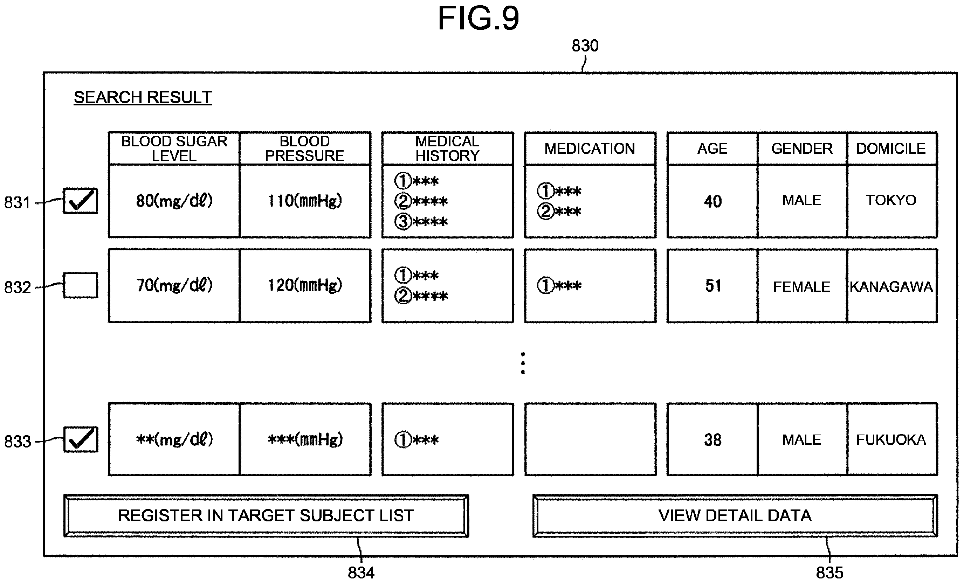

FIG. 9 is a diagram illustrating an example of a search result screen displayed by the research institution terminal 50. As illustrated in FIG. 9, a search result screen 830 displayed by the research institution terminal 50 includes vital data (in FIG. 9 "blood sugar level" and "blood pressure" appear as an example), attribute data, and setting data (in FIG. 9 "medical history", "medication", "age", "gender", and "domicile" appear as an example) as the search result based on the search button 823 (FIG. 8) pressed. The search result screen 830 may have the sorting function for each item.

The search result screen 830 also includes data check boxes 831 to 833. In the example illustrated in FIG. 9, a check mark appears in the data check box 831 and the data check box 833 to indicate that data has been selected. When a target subject list register button 834 is pressed, the provider corresponding to the selected data is registered in a target subject list. On the other hand, when a detail data view button 835 is pressed, a detail data screen corresponding to the selected data appears.

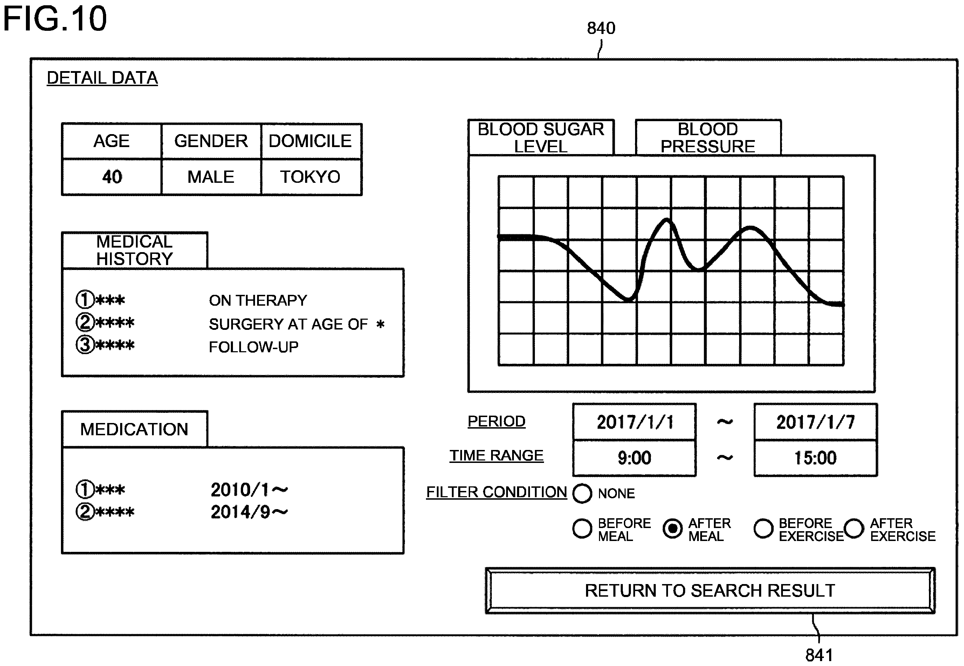

FIG. 10 is a diagram illustrating an example of the detail data screen displayed by the research institution terminal 50. As illustrated in FIG. 10, a detail data screen 840 displayed by the research institution terminal 50 includes age, gender, and domicile as well as the detail of medical history, and the detail of medication. The detail data screen 840 also includes temporal change for each item of vital data (in the example illustrated in FIG. 10, blood sugar level and blood pressure).

As illustrated in FIG. 10, a period and a time range for the temporal change of vital data can be specified. This enables the user to examine not only a long-term change of vital data but also a short-term change (for example, change for one day). As illustrated in FIG. 10, a filter condition of vital data can also be specified. Specifically, any one of the filter conditions "none", "before meal", "after meal", "before exercise", and "after exercise" can be specified so that vital data narrowed down by the specified filter condition appears. When a return button 841 is pressed, the display is controlled to return to the search result screen 830.

In this way, the biological information provided by the provider P1 is used in whole or in part by the research institution 55 (user). The research institution 55 may be tempted to ask the provider P1 for cooperation on research while the research institution 55 is using the biological information provided by the provider P1 in whole or in part. In such a case, a consideration may be paid from the research institution 55 to the organizer of the data management system 20 for the success in matching between the provider P1 and the research institution 55. The organizer of the data management system 20 thus can enjoy the merit.

More specifically, when it is detected that the provider P1 will cooperate on clinical research conducted by the research institution 55 (user) (for example, when that the provider P1 will cooperate is input by the provider P1, transmitted from the provider terminal 10, and received by the data management system 20), the determination unit 214 determines a contingency fee to be paid by the research institution 55. The contingency fee may be, but not limited to, the amount of money obtained by multiplying the price paid by the research institution 55 for the cooperation by the provider P1 by a predetermined ratio (for example, a small percent). The processing unit 216 may perform a settlement process of withdrawing the contingency fee from the account of the research institution 55.

The second embodiment of the present disclosure has been described above.

<3. Third Embodiment>

A third embodiment of the present disclosure will now be described.

[3.1. System Configuration Example]

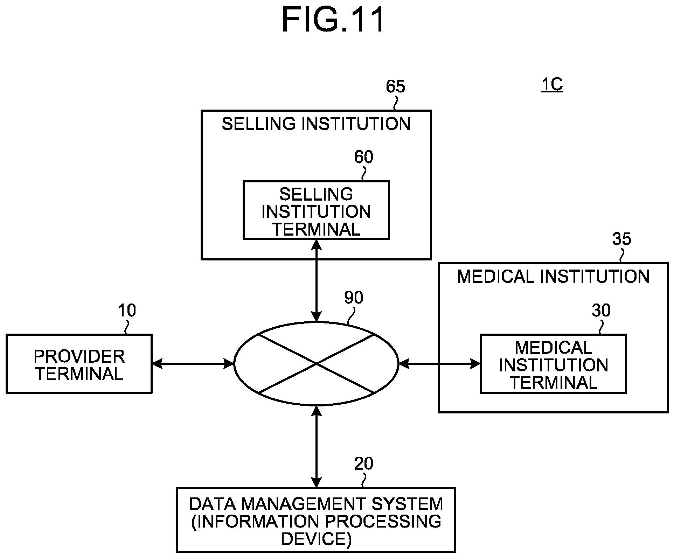

A configuration example of an information processing system 1C according to the third embodiment of the present disclosure will now be described. FIG. 11 is a diagram illustrating a configuration example of the information processing system 1C according to the third embodiment of the present disclosure. As illustrated in FIG. 11, the information processing system 1C according to the third embodiment of the present disclosure differs from the information processing system 1A according to the first embodiment of the present disclosure in that it has a selling institution terminal 60 instead of the insurance management terminal 40.

The selling institution terminal 60 is used in a selling institution 65. The selling institution 65 may be an institution that provides products or services (specifically, an institution that aims to gain a profit by supporting personal healthcare), and examples include medical institutions, pharmaceutical companies, and sports gyms. In the configuration of the information processing system 1C according to the third embodiment of the present disclosure, the same configuration as the configuration of the information processing system 1A according to the first embodiment of the present disclosure will not be further elaborated, and a different configuration is mainly described below.

A configuration example of the information processing system 1C according to the third embodiment of the present disclosure has been described above.

[3.2. Detail of Functions of Information Processing System]

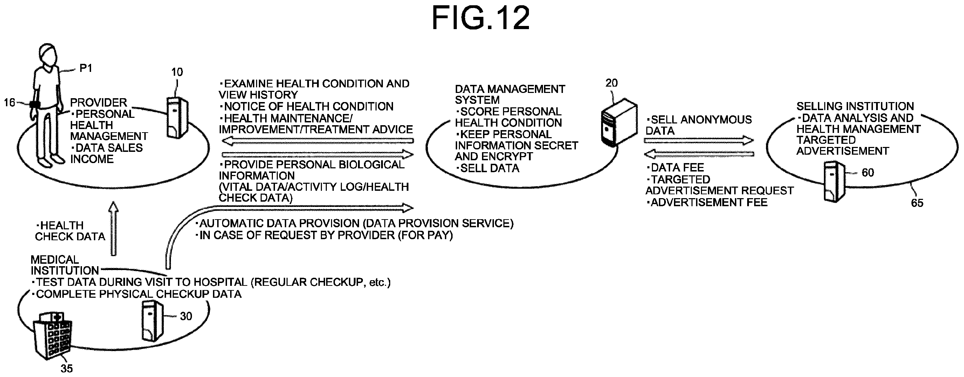

The detail of functions of the information processing system 1C according to the third embodiment of the present disclosure will now be described. FIG. 12 is a diagram for explaining the overall functions of the information processing system 1C according to the third embodiment of the present disclosure. As illustrated in FIG. 12, the third embodiment of the present disclosure differs from the first embodiment of the present disclosure in that the selling institution terminal 60 used in the selling institution 65 is present instead of the insurance management terminal 40 used in the insurance management institution 45.

As described in the first embodiment of the present disclosure, the data acquisition unit 212 acquires information related to biological information. The determination unit 214 determines a reward value provided to the provider P1 as feedback for provision of biological information, based on information related to biological information. The processing unit 216 performs a predetermined process according to the reward value.

Also in the third embodiment of the present disclosure, it is mainly assumed that the reward value is the amount of money in the same manner as in the first embodiment of the present disclosure. Specifically, in the third embodiment of the present disclosure, it is assumed that the selling institution 65 makes a request to present a targeted advertisement (for example, through the data management system 20 as illustrated in FIG. 12), and the provider P1 provides biological information as biological information to be used in data analysis for targeted advertisement presentation in the selling institution 65.

The provider P1 provides biological information as the biological information to be used for data analysis, whereby the selling institution 65 can search for desired biological information in chronological order (for example, for pay) in addition to age, gender, established disease, and drug usage and present a targeted advertisement appropriate for the provider P1 (for example, a targeted advertisement is transmitted from the selling institution terminal 60 to the provider terminal 10).

For example, the selling institution 65 searches for a provider serving as a target through data analysis and additionally pays an advertising fee to present advertisements related to products or services or issue coupons. This process creates an awareness to a provider who notices few symptoms and attracts a new customer. Since a more appropriate targeted advertisement can be presented to the provider, the cost effectiveness for advertising fees can be increased.

Traditionally, techniques called targeting advertisement have been used, which analyze the user's statement in a social networking service (SNS) and the like. However, since the user's statement is controlled by the user's own will, the analysis of statement may fail to find a user serving as a desirable target. On the other hand, as in the third embodiment of the present disclosure, biological information can be used to find a target user more appropriately.

In such a case, in the third embodiment of the present disclosure, it is mainly assumed that the reward value is the amount of money (data analysis cooperation fee) paid to the provider P1 for cooperation on data analysis.

The information related to biological information may include the data size of biological information provided by the provider P1 (may be pay-per-use), may include the number of items that constitute the biological information provided by the provider P1, or may include the period in which the biological information provided by the provider P1 is provided (for example, per month when a monthly use agreement is made, or per year when an annual use agreement is made). The determination unit 214 determines a data analysis cooperation fee paid to the provider P1, based on the information related to biological information. The processing unit 216 may perform a settlement process of transferring the data analysis cooperation fee to the account of the provider P1.

The data analysis cooperation fee is transmitted by the communication unit 240 to the selling institution terminal 60 and displayed by the selling institution terminal 60. The data analysis cooperation fee is transmitted by the communication unit 240 to the provider terminal 10 and displayed by the provider terminal 10.

For example, it is thought that the larger the data size of biological information provided by the provider P1 is, the larger the contribution by the provider P1 to data analysis is. Accordingly, the larger the data size of biological information provided by the provider P1 is, the higher data analysis cooperation fee provided to the provider P1 may be set by the determination unit 214. If so, it is expected that the provider P1 is motivated to provide biological information of a larger data size.

It is thought that the larger the number of items that constitute the biological information provided by the provider P1 is, the larger the contribution by the provider P1 to data analysis is. Accordingly, the larger the number of items that constitute biological information provided by the provider P1 is, the higher data analysis cooperation fee paid to the provider P1 may be set by the determination unit 214. If so, it is expected that the provider P1 is motivated to provide biological information including more items.

It is thought that the longer the period in which the biological information provided by the provider P1 is provided, the larger the contribution by the provider P1 to data analysis is. The longer the period in which biological information is provided by the provider P1 is, the higher research cooperation fee for the provider P1 may be set by the determination unit 214. If so, it is expected that the provider P1 is motivated to provide biological information for a longer period.

The data analysis cooperation fee may be fixed or may vary depending on situations. For example, when the provider P1 accepts receiving advertisement presentation, the determination unit 214 may determine the data analysis cooperation fee provided to the provider P1 to be high compared with when the provider P1 rejects receiving advertisement presentation. If so, the provider P1 is encouraged to accept receiving advertisement presentation, and it is more likely that advertisements promote sales of products or services.

The data usage in the selling institution 65 will be described. A search screen displayed by the selling institution terminal 60 may be similar to the search screen 810 (FIG. 8) described in the second embodiment of the present disclosure. When the search button 823 is pressed in the search screen 810 (FIG. 8), vital data that satisfies these conditions is retrieved from vital data provided from one or more providers.

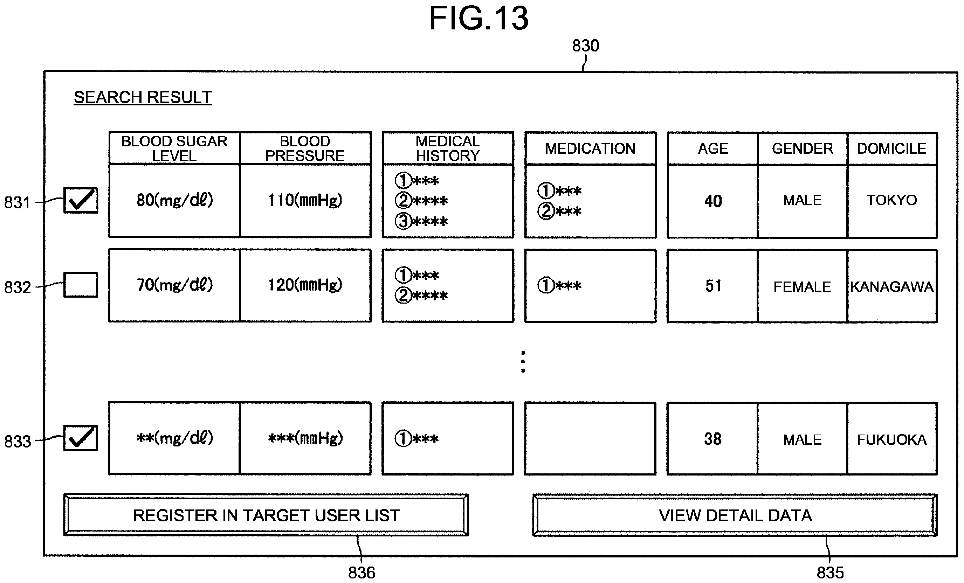

FIG. 13 is a diagram illustrating an example of a search result screen displayed by the selling institution terminal 60. As illustrated in FIG. 13, the search result screen 830 displayed by the selling institution terminal 60 differs from the search result screen 830 (FIG. 9) described in the second embodiment of the present disclosure in that it includes a target user list register button 836 instead of the target subject list register button 834. When the target user list register button 836 is pressed, the provider corresponding to the selected data is registered in the target user list. On the other hand, when the detail data view button 835 is pressed, a detail data screen corresponding to the selected data appears.

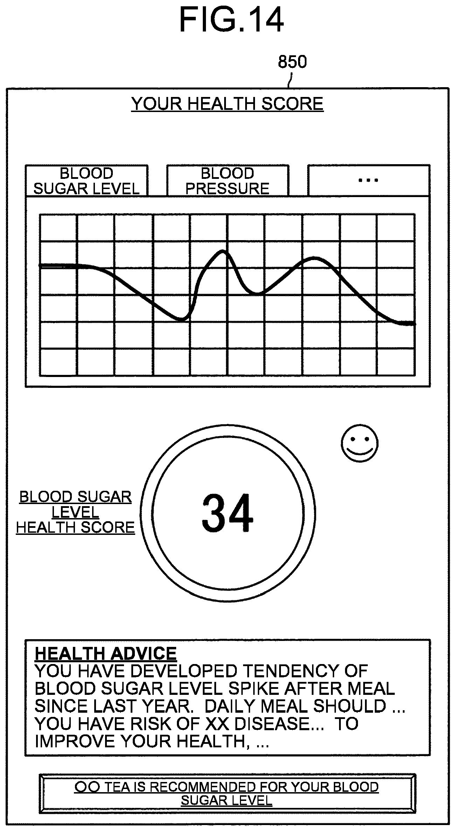

When an advertisement to be presented to the target user is determined by the selling institution 65, the advertisement is transmitted from the selling institution terminal 60 to the provider terminal 10, and the advertisement (advertisement screen) is displayed by the provider terminal 10. FIG. 14 is a diagram illustrating an example of the advertisement screen displayed by the provider terminal 10. As illustrated in FIG. 14, an advertisement screen 850 displayed by the provider terminal 10 includes one or more items that constitute vital data (in the example illustrated in FIG. 14, "blood sugar level", "blood pressure"). When an item is selected, the temporal change of vital data and the health score corresponding to the selected item appear.

In the example illustrated in FIG. 14, the advertisement screen 850 includes the temporal change and the health score "34" of vital data corresponding to the item "blood sugar level". However, the temporal change and the health score of vital data corresponding to each item may be displayed in the form of a list. The longer period of vital data is displayed according to the provision period of vital data, so that the transition of vital data can be examined retroactively in the advertisement screen 850.

As illustrated in FIG. 14, the advertisement screen 850 may include advice on health (health advice). For example, as illustrated in FIG. 14, the health advice may include the result of data analysis conducted for the displayed health score of vital data by the data management system 20 or the selling institution 65. As illustrated in FIG. 14, the health advice may include the risk of suffering a disease and advice such as proposal of a risk improving method.

As illustrated in FIG. 14, the selling institution 65 may include an advertisement related to its product (or serve) and a link to its site in the advertisement screen 850. Alternatively, the selling institution 65 may include a purchase button for its product (or service) in the advertisement screen 850.

In this way, the biological information provided by the provider P1 is used in whole or in part (for example, as anonymous data) for data analysis in the selling institution 65. In this way, when there is a user (selling institution 65) of the biological information provided by the provider P1 in whole or in part, a data usage fee may be provided from the selling institution 65 to the data management system 20. In this case, the determination unit 214 determines the amount of money (data usage fee) to be paid by the selling institution 65, based on information related to biological information that is used by the user in the biological information provided by the provider P1.

The information related to the biological information used by the selling institution 65 (user) may include the data size of the biological information used by the selling institution 65, may include the number of items that constitute the biological information used by the selling institution 65, or may include the period in which biological information is used by the selling institution 65. The determination unit 214 determines a data usage fee, based on the information related to the biological information used by the selling institution 65. The processing unit 216 may perform a settlement process of withdrawing the data usage fee from the account of the selling institution 65.

The data usage fee is transmitted by the communication unit 240 to the selling institution terminal 60 and displayed by the selling institution terminal 60. The data usage fee is transmitted by the communication unit 240 to the provider terminal 10 and displayed by the provider terminal 10.

The data usage in the selling institution 65 will be described. The selling institution terminal 60 determines an advertisement to be presented to the provider P1 from among advertisements related to products or services sold by the selling institution 65 (user), based on the information related to biological information.

The provider P1 may be tempted to purchase a product or service related to the advertisement while viewing the presented advertisement. In such a case, a consideration may be paid from the selling institution 65 to the organizer of the data management system 20 for the success in matching between the provider P1 and the selling institution 65 (user). The organizer of the data management system 20 thus can enjoy the merit.

More specifically, when it is detected that the provider P1 has purchased a product or service related to the advertisement presented by the selling institution 65 (user) to the provider P1 (for example, when to purchase the product or service is input by the provider P1, transmitted from the provider terminal 10, and received by the data management system 20), the determination unit 214 determines a contingency fee to be paid by the selling institution 65. The contingency fee may be, but not limited to, the amount of money obtained by multiplying the price of the product or service purchased by the provider P1 by a predetermined ratio (for example, a small percent). The processing unit 216 may perform a settlement process of withdrawing the contingency fee from the account of the selling institution 65.

The case where an advertisement to be presented to the provider P1 is determined from among advertisements related to products or services sold by the selling institution 65 (user) based on the information related to biological information has mainly been described above. However, a medical institution may be present as the user of biological information, instead of the selling institution 65. In such a case, the medical institution may present advice (for example, charge-free advice) to the provider P1 instead of an advertisement (for example, advice may be transmitted from the medical institution terminal to the provider terminal 10). The advice presented to the provider P1 may be determined by the medical institution based on the information related to biological information, in the same manner as the advertisement.

The third embodiment of the present disclosure has been described above.

<4.Hardware Configuration Example>

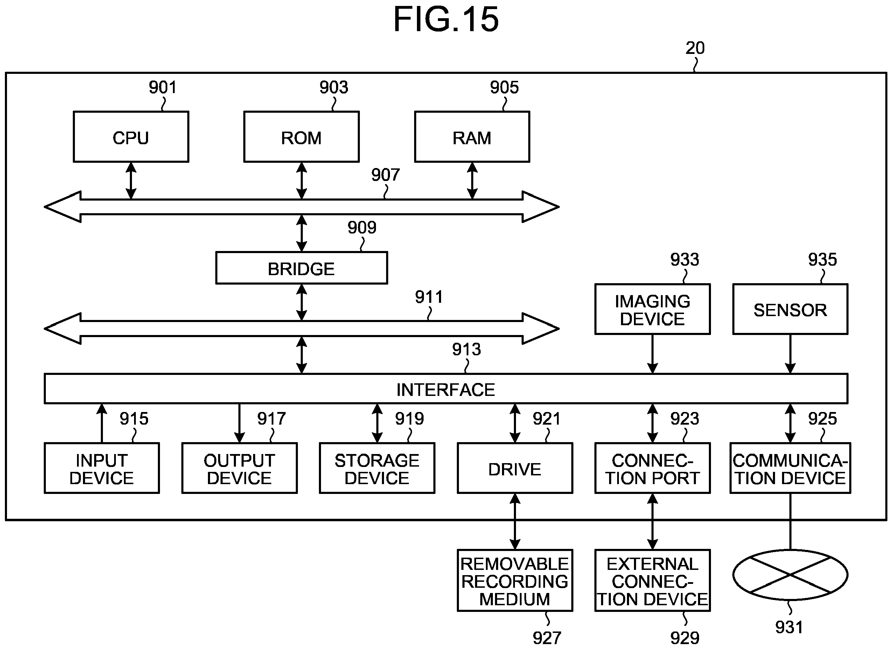

Referring now to FIG. 15, a hardware configuration of the data management system 20 according to the embodiments of the present disclosure will be described. FIG. 15 is a block diagram illustrating a hardware configuration example of the data management system 20 according to the embodiments of the present disclosure. Although a hardware configuration example of the data management system 20 is described here, the hardware configuration of a variety of terminals (for example, the provider terminal 10, the medical institution terminal 30, the insurance management terminal 40, the research institution terminal 50, the selling institution terminal 60) may also be implemented in the same manner as the hardware configuration example of the data management system 20 described here.

As illustrated in FIG. 15, the data management system 20 includes a central processing unit (CPU) 901, a read only memory (ROM) 903, and a random access memory (RAM) 905. The data management system 20 may also include a host bus 907, a bridge 909, an external bus 911, an interface 913, an input device 915, an output device 917, a storage device 919, a drive 921, a connection port 923, and a communication device 925. The data management system 20 may further include an imaging device 933 and a sensor 935 if necessary. The data management system 20 may have a processing circuit called a digital signal processor (DSP) or an application specific integrated circuit (ASIC), instead of or in addition to the CPU 901.

The CPU 901 functions as an arithmetic processing unit and a control device and controls the entire operation or part of the operation in the data management system 20 under instructions of computer programs stored in the ROM 903, the RAM 905, the storage device 919, or a removable recording medium 927. The ROM 903 stores computer programs or arithmetic operation parameters to be used by the CPU 901. The RAM 905 temporarily stores a computer program used in execution of the CPU 901 and parameters changing as appropriate in the execution. The CPU 901, the ROM 903, and the RAM 905 are connected with each other through the host bus 907 configured with an internal bus such as a CPU bus. The host bus 907 is further connected to the external bus 911 such as a peripheral component interconnect/interface (PCI) bus through the bridge 909.

The input device 915 is a device operated by a user, such as a button. The input device 915 may include a mouse, a keyboard, a touch panel, a switch, and a lever. The input device 915 may include a microphone detecting user's voice. The input device 915 may be, for example, a remote controller using infrared radiation or other radio waves or may be an external connection device 929, such as a mobile phone, adapted to operate the data management system 20. The input device 915 includes an input control circuit that generates an input signal based on information input by the user and outputs the generated signal to the CPU 901. The user operates the input device 915 to input a variety of data or a processing operation instruction to the data management system 20. The imaging device 933 described later may also function as an input device by imaging the motion of the user's hand or the user's finger. In this case, the pointing position may be determined depending on the motion of the hand or the direction of the finger.

The output device 917 is configured with a device that can give the acquired information to the user visually or acoustically. The output device 917 may be, for example, a display device such as a liquid crystal display (LCD) and an organic electro-luminescence (EL) display or a sound output device such as a speaker and headphones. The output device 917 may include a plasma display panel (PDP), a projector, a hologram, and a printer. The output device 917 outputs the result obtained by the processing by the data management system 20 in the form of image such as text or picture or in the form of sound such as voice or audio. The output device 917 may include a light for illuminating the surrounding.

The storage device 919 is a device for data storage configured as an example of the storage unit of the data management system 20. The storage device 919 is configured with, for example, a magnetic storage device such as a hard disk drive (HDD), a semiconductor storage device, an optical storage device, or a magnetic optical storage device. This storage device 919 stores a computer program and a variety of data executed by the CPU 901, and a variety of data externally acquired.

The drive 921 is a reader/writer for the removable recording medium 927 such as a magnetic disc, an optical disc, a magnetic optical disc, or a semiconductor memory and is contained in the data management system 20 or externally attached. The drive 921 reads information recorded on the attached removable recording medium 927 and outputs the read information to the RAM 905. The drive 921 also writes a record into the attached removable recording medium 927.

The connection port 923 is a port for directly connecting a device to the data management system 20. The connection port 923 may be, for example, a universal serial bus (USB) port, an IEEE1394 port, or a small computer system interface (SCSI) port. The connection port 923 may be, for example, an RS-232C port, an optical audio terminal, a high-definition multimedia interface (HDMI) (registered trademark) port. The external connection device 929 is connected to the connection port 923 so that a variety of data can be exchanged between the data management system 20 and the external connection device 929.

The communication device 925 is a communication interface configured with, for example, a communication device for connecting to a network 931. The communication device 925 may be, for example, a communication card for a wired or wireless local area network (LAN), Bluetooth (registered trademark), or wireless USB (WUSB). The communication device 925 may be a router for optical communication, a router for asymmetric digital subscriber line (ADSL), or a variety of communication modems. The communication device 925 transmits/receives, for example, signals to/from the Internet or other communication devices using a predetermined protocol such as TCP/IP. The network 931 connected to the communication device 925 is a network connected by wire or wirelessly, and examples include the Internet, home LAN, infrared communication, radio wave communication, and satellite communication.

The imaging device 933 is a device that images the real space, for example, using an imager such as charge coupled device (CCD) or complementary metal oxide semiconductor (CMOS) and a variety of members such as a lens for controlling image formation of a subject image on the imager, and generates a captured image. The imaging device 933 may capture still images or may capture moving images.

The sensor 935 is a variety of sensors such as a distance-measurement sensor, an acceleration sensor, a gyro sensor, a geomagnetic sensor, a vibration sensor, an optical sensor, and an acoustic sensor. The sensor 935 acquires, for example, information about a state of the data management system 20 per se, such as the posture of the casing of the data management system 20, and information about the environment surrounding the data management system 20, such as brightness and noise in the surroundings of the data management system 20. The sensor 935 may include a global positioning system (GPS) sensor that receives GPS signals to measure the latitude, longitude, and altitude of the device.

<5. Closing>

As explained above, embodiments of the present disclosure provide the data management system 20 which includes the data acquisition unit 212 configured to acquire information related to biological information provided by the provider P1 and the determination unit 214 configured to determine the reward value provided to the provider P1 as feedback for provision of biological information, based on the information related to biological information.

Such a configuration can improve the motivation of the provider P1 of biological information for healthcare. In addition, such a configuration encourages the provider P1 to provide biological information, and it is expected that the biological information by the provider P1 is utilized more efficiently.

Although preferred embodiments of the present disclosure have been described in detail above with reference to the accompanying drawings, the technical scope of the present disclosure is not limited by such embodiments. One having ordinary knowledge in the technical field of the present disclosure would conceive a variety of changes or modifications without departing from the technical concept described in the claims, and it should be understood that these changes and modifications fall within the technical scope of the present disclosure.

For example, in the foregoing embodiments, the data management system 20 is provided independently of the other terminals. However, any other terminal may have some or all of the functions of the data management system 20. Alternatively, some or all of the functions of the data management system 20 may be distributed over a plurality of terminals.

The effect described in the present description is only explanatory or illustrative and is not intended to be limitative. The technique according to the present disclosure may achieve other effects apparent to those skilled in the art from the disclosure in the present description, in addition to the effect above or instead of the effect above.

The following configuration may also fall within the technical scope of the present disclosure.

(1) An information processing device comprising:

a data acquisition unit configured to acquire information related to biological information, the biological information being provided by a provider; and

a determination unit configured to determine a reward value provided to the provider as feedback for provision of the biological information, based on the information related to biological information.

(2) The information processing device according to (1), wherein the information related to biological information includes a calculation result calculated based on the biological information.

(3) The information processing device according to (2), wherein the calculation result is calculated based on at least one of an evaluation value of the biological information, a period in which the biological information is kept in a predetermined range, a tendency of change in the biological information, the amount of physical activity of the provider when the biological information is detected, and a confidence of the biological information.

(4) The information processing device according to (3), wherein the confidence is calculated based on at least one of the number of samples of the biological information, a sampling period of the biological information, and an average sampling interval of the biological information.

(5) The information processing device according to any one of (1) to (4), wherein when there are a plurality of items that constitute the biological information, the information related to biological information includes a calculation result calculated based on a calculation result and a weight for each item.

(6) The information processing device according to (5), wherein the weight is calculated based on at least one of the amount of physical activity of the provider and a confidence of the biological information.

(7) The information processing device according to any one of (1) to (6), wherein the information related to biological information includes at least one of a data size of the biological information provided by the provider, the number of items that constitute the biological information, and a period in which the biological information is provided.

(8) The information processing device according to any one of (1) to (7), wherein the information processing device includes a processing unit configured to execute a predetermined process according to the reward value.

(9) The information processing device according to (8), wherein the processing unit calculates an insurance premium to be paid by the provider by deducting the reward value from an insurance premium as a basis.

(10) The information processing device according to any one of (1) to (9), wherein when there is a user of the biological information in whole or in part, the determination unit determines a data usage fee to be paid by the user, based on information related to biological information used by the user.

(11) The information processing device according to (10), wherein the information related to biological information used by the user includes at least one of a data size of the biological information, the number of items that constitute the biological information, and a period in which the biological information is used.

(12) The information processing device according to (10) or (11), wherein when it is detected that the provider cooperates on a research conducted by the user, the determination unit determines a contingency fee to be paid by the user.

(13) The information processing device according to any one of (10) to (12), wherein an advertisement to be presented to the provider is determined from among advertisements related to a product or a service sold by the user, based on the information related to biological information.

(14) The information processing device according to (13), wherein when it is detected that the product or the service has been purchased by the provider, the determination unit determines a contingency fee to be paid by the user.

(15) The information processing device according to (13) or (14), wherein when receiving the advertisement is accepted, the determination unit determines the reward value provided to the provider to be high, compared with when receiving the advertisement is rejected.

(16) The information processing device according to any one of (10) to (15), wherein advice presented by the user to the provider is determined based on the information related to biological information.

(17) The information processing device according to any one of (1) to (16), wherein the biological information includes at least one of heart rate, blood pressure, blood sugar level, breathing rate, body temperature, brain wave, electrocardiogram, blood flow, cardiac sound, blood oxygen level, cholesterol, blood-alcohol level, perspiration, myogram, and calories burned of the provider.

(18) The information processing device according to any one of (1) to (17), wherein the reward value includes at least one of an amount of money and a point having a value equivalent to the amount of money.

(19) An information processing method comprising:

acquiring information related to biological information, the biological information being provided by a provider; and determining a reward value provided to the provider as feedback for provision of the biological information, based on the information related to biological information.

(20) A computer program causing a computer to function as an information processing device comprising:

a data acquisition unit configured to acquire information related to biological information, the biological information being provided by a provider; and

a determination unit configured to determine a reward value provided to the provider as feedback for provision of the biological information, based on the information related to biological information.

Reference Signs List

| | |
|---|---|
| 1 (1A to 1C) | information processing system |
| 10 | provider terminal |
| 16 | wearable device |
| 20 | data management system |
| 30 | medical institution terminal |
| 35 | medical institution |
| 40 | insurance management terminal |
| 45 | insurance management institution |
| 50 | research institution terminal |
| 55 | research institution |
| 60 | selling institution terminal |
| 65 | selling institution |
| 210 | control unit |
| 212 | data acquisition unit |
| 214 | determination unit |
| 216 | processing unit |
| 220 | operation unit |
| 230 | storage unit |
| 240 | communication unit |
| 250 | output unit |

**Claims**

1. An information processing device comprising:

   a data acquisition unit configured to acquire information related to biological information, the biological information being provided by a provider; and
   a determination unit configured to determine a reward value provided to the provider as feedback for provision of the biological information, based on the information related to biological information.

2. The information processing device according to claim 1, wherein the information related to biological information includes a calculation result calculated based on the biological information.

3. The information processing device according to claim 2, wherein the calculation result is calculated based on at least one of an evaluation value of the biological information, a period in which the biological information is kept in a predetermined range, a tendency of change in the biological information, the amount of physical activity of the provider when the biological information is detected, and a confidence of the biological information.

4. The information processing device according to claim 3, wherein the confidence is calculated based on at least one of the number of samples of the biological information, a sampling period of the biological information, and an average sampling interval of the biological information.

5. The information processing device according to claim 1, wherein when there are a plurality of items that constitute the biological information, the information related to biological information includes a calculation result calculated based on a calculation result and a weight for each item.

6. The information processing device according to claim 5, wherein the weight is calculated based on at least one of the amount of physical activity of the provider and a confidence of the biological information.

**7.** The information processing device according to claim 1, wherein the information related to biological information includes at least one of a data size of the biological information provided by the provider, the number of items that constitute the biological information, and a period in which the biological information is provided.

**8.** The information processing device according to claim 1, wherein the information processing device includes a processing unit configured to execute a predetermined process according to the reward value.

**9.** The information processing device according to claim 8, wherein the processing unit calculates an insurance premium to be paid by the provider by deducting the reward value from an insurance premium as a basis.

**10.** The information processing device according to claim 1, wherein when there is a user of the biological information in whole or in part, the determination unit determines a data usage fee to be paid by the user, based on information related to biological information used by the user.

**11.** The information processing device according to claim 10, wherein the information related to biological information used by the user includes at least one of a data size of the biological information, the number of items that constitute the biological information, and a period in which the biological information is used.

**12.** The information processing device according to claim 10, wherein when it is detected that the provider cooperates on a research conducted by the user, the determination unit determines a contingency fee to be paid by the user.

**13.** The information processing device according to claim 10, wherein an advertisement to be presented to the provider is determined from among advertisements related to a product or a service sold by the user, based on the information related to biological information.

**14.** The information processing device according to claim 13, wherein when it is detected that the product or the service has been purchased by the provider, the determination unit determines a contingency fee to be paid by the user.

**15.** The information processing device according to claim 13, wherein when receiving the advertisement is accepted, the determination unit determines the reward value provided to the provider to be high, compared with when receiving the advertisement is rejected.

**16.** The information processing device according to claim 10, wherein advice presented by the user to the provider is determined based on the information related to biological information.

**17.** The information processing device according to claim 1, wherein the biological information includes at least one of heart rate, blood pressure, blood sugar level, breathing rate, body temperature, brain wave, electrocardiogram, blood flow, cardiac sound, blood oxygen level, cholesterol, blood-alcohol level, perspiration, myogram, and calories burned of the provider.

**18.** The information processing device according to claim 1, wherein the reward value includes at least one of an amount of money and a point having a value equivalent to the amount of money.

**19.** An information processing method comprising:

acquiring information related to biological information, the biological information being provided by a provider; and determining a reward value provided to the provider as feedback for provision of the biological information, based on the information related to biological information.

**20.** A computer program causing a computer to function as an information processing device comprising:

a data acquisition unit configured to acquire information related to biological information, the biological information being provided by a provider; and a determination unit configured to determine a reward value provided to the provider as feedback for provision of the biological information, based on the information related to biological information.

# FIG.1

1A

```
            45
    ┌─────────────────────┐
    │     INSURANCE        │
    │   MANAGEMENT         │
    │   INSTITUTION        │
    │            40        │
    │   ┌─────────────┐    │
    │   │  INSURANCE  │    │
    │   │ MANAGEMENT  │    │
    │   │  TERMINAL   │    │
    │   └─────────────┘    │
    └─────────────────────┘
```

                                                    35
                                    ┌──────────────────────────┐
                                    │   MEDICAL INSTITUTION     │
                                    │               30          │
                                    │   ┌──────────────┐        │
                                    │   │   MEDICAL    │        │
                                    │   │ INSTITUTION  │        │
                                    │   │   TERMINAL   │        │
                                    │   └──────────────┘        │
                                    └──────────────────────────┘

        10                 90
┌──────────────┐
│   PROVIDER   │
│   TERMINAL   │
└──────────────┘

                    20
┌──────────────────────────────┐
│   DATA MANAGEMENT SYSTEM      │
│  (INFORMATION PROCESSING      │
│          DEVICE)              │
└──────────────────────────────┘

# FIG.2

DATA MANAGEMENT SYSTEM (INFORMATION PROCESSING DEVICE) 20

OPERATION UNIT 220

COMMUNICA-TION UNIT 240

CONTROL UNIT 210

DATA ACQUISITION UNIT 212

DETERMINA-TION UNIT 214

PROCESSING UNIT 216

STORAGE UNIT 230

OUTPUT UNIT 250

EP 3 657 430 A1

# FIG.3

LOCAL GOVERNMENT
(MINISTRY OF HEALTH, LABOUR, AND WELFARE)
· HEALTH INSURANCE PREMIUM DISCOUNT ACCORDING TO FUTURE MEDICAL EXPENSE

40

45

P1

16

10

· EXAMINE HEALTH CONDITION AND VIEW HISTORY
· NOTICE OF HEALTH CONDITION
· HEALTH INSURANCE PREMIUM DISCOUNT

PROVIDER
· PERSONAL HEALTH MANAGEMENT
· DATA SALES INCOME

DATA MANAGEMENT SYSTEM
· SCORE PERSONAL HEALTH CONDITION
· PREDICT FUTURE MEDICAL EXPENSE
· KEEP PERSONAL INFORMATION SECRET AND ENCRYPT

20

· PROVIDE PERSONAL BIOLOGICAL INFORMATION
(VITAL DATA/ACTIVITY LOG/HEALTH CHECK DATA)

· HEALTH CHECK DATA

· AUTOMATIC DATA PROVISION (DATA PROVISION SERVICE)
· IN CASE OF REQUEST BY PROVIDER (FOR PAY)

MEDICAL INSTITUTION
· TEST DATA DURING VISIT TO HOSPITAL (REGULAR CHECKUP, etc.)
· COMPLETE PHYSICAL CHECKUP DATA

30

35

EP 3 657 430 A1

# FIG.4

Alo          Ahi

# FIG.5

DETERIORATING
TENDENCY

Ahi

IMPROVING
TENDENCY

IMPROVING
TENDENCY

Alo

DETERIORATING
TENDENCY

# FIG.6

# FIG.7

PROVIDER
·PERSONAL HEALTH MANAGEMENT
·DATA SALES INCOME
·CLINICAL TEST COOPERATION INCOME

·EXAMINE HEALTH CONDITION AND VIEW HISTORY
·NOTICE OF HEALTH CONDITION
·REQUEST FOR CLINICAL RESEARCH COOPERATION (FOR PAY)

·PROVIDE PERSONAL BIOLOGICAL INFORMATION (VITAL DATA/ACTIVITY LOG/HEALTH CHECK DATA)

DATA MANAGEMENT SYSTEM
·SCORE PERSONAL HEALTH CONDITION
·KEEP PERSONAL INFORMATION SECRET AND ENCRYPT
·SELL DATA

·HEALTH CHECK DATA

·AUTOMATIC DATA PROVISION (DATA PROVISION SERVICE)
·IN CASE OF REQUEST BY PROVIDER (FOR PAY)

·SELL ANONYMOUS DATA FOR CLINICAL RESEARCH

·DATA FEE
·REQUEST FOR CLINICAL RESEARCH COOPERATION

MEDICAL INSTITUTION
·TEST DATA DURING VISIT TO HOSPITAL (REGULAR CHECKUP, etc.)
·COMPLETE PHYSICAL CHECKUP DATA

RESEARCH INSTITUTION
·UTILIZE FOR CLINICAL DATA FOR TREATMENT AND DRUG DISCOVERY

EP 3 657 430 A1

# FIG.8

EP 3 657 430 A1

VITAL DATA

| | VALUE | | | PERIOD | |
|---|---|---|---|---|---|
| BLOOD SUGAR LEVEL ▼ | ** | ~ | ** | 2010/1/1 | ~ | 2017/1/1 |

812 — and ▼

| BLOOD PRESSURE ▼ | ** | ~ | ** | ****/**/** | ~ | ****/**/** |

⋮

| ** ▼ | ** | ~ | ** | ****/**/** | ~ | ****/**/** |

+
ADD

MEDICAL HISTORY

| ** ▼ |
|---|

⋮

| ** ▼ |
|---|

+
ADD

MEDICATION

| ** ▼ |
|---|

⋮

| ** ▼ |
|---|

+
ADD

| | AGE | | | GENDER | | DOMICILE | | | |
|---|---|---|---|---|---|---|---|---|---|
| 40 | ~ | 49 | | MALE ▼ | | TOKYO ▼ | | | SEARCH |

# FIG.9

SEARCH RESULT

| | BLOOD SUGAR LEVEL | BLOOD PRESSURE | MEDICAL HISTORY | MEDICATION | AGE | GENDER | DOMICILE |
|---|---|---|---|---|---|---|---|
| ☑ 831 | 80(mg/dℓ) | 110(mmHg) | ①*** ②**** ③**** | ①*** ②*** | 40 | MALE | TOKYO |
| ☐ 832 | 70(mg/dℓ) | 120(mmHg) | ①*** ②**** | ①*** | 51 | FEMALE | KANAGAWA |
| ⋮ | | | | | | | |
| ☑ 833 | **(mg/dℓ) | ***(mmHg) | ①*** | | 38 | MALE | FUKUOKA |

| REGISTER IN TARGET SUBJECT LIST | VIEW DETAIL DATA |
|---|---|

834

835

830

EP 3 657 430 A1

# FIG.10

**DETAIL DATA** — 840

| AGE | GENDER | DOMICILE |
|---|---|---|
| 40 | MALE | TOKYO |

**MEDICAL HISTORY**

①***     ON THERAPY
②****     SURGERY AT AGE OF *
③****     FOLLOW-UP

**MEDICATION**

①***     2010/1～
②****     2014/9～

| BLOOD SUGAR LEVEL | BLOOD PRESSURE |
|---|---|

PERIOD    2017/1/1 ～ 2017/1/7

TIME RANGE    9:00 ～ 15:00

FILTER CONDITION ○ NONE

○ BEFORE MEAL   ● AFTER MEAL   ○ BEFORE EXERCISE   ○ AFTER EXERCISE

**RETURN TO SEARCH RESULT** — 841

EP 3 657 430 A1

# FIG.11

# FIG.12

- EXAMINE HEALTH CONDITION AND VIEW HISTORY
- NOTICE OF HEALTH CONDITION
- HEALTH MAINTENANCE/ IMPROVEMENT/TREATMENT ADVICE

PROVIDER
- PERSONAL HEALTH MANAGEMENT
- DATA SALES INCOME

- PROVIDE PERSONAL BIOLOGICAL INFORMATION
(VITAL DATA/ACTIVITY LOG/HEALTH CHECK DATA)

DATA MANAGEMENT SYSTEM
- SCORE PERSONAL HEALTH CONDITION
- KEEP PERSONAL INFORMATION SECRET AND ENCRYPT
- SELL DATA

- SELL ANONYMOUS DATA

- DATA FEE
- TARGETED ADVERTISEMENT REQUEST
- ADVERTISEMENT FEE

SELLING INSTITUTION
- DATA ANALYSIS AND HEALTH MANAGEMENT TARGETED ADVERTISEMENT

- HEALTH CHECK DATA

- AUTOMATIC DATA PROVISION (DATA PROVISION SERVICE)
- IN CASE OF REQUEST BY PROVIDER (FOR PAY)

MEDICAL INSTITUTION
- TEST DATA DURING VISIT TO HOSPITAL (REGULAR CHECKUP, etc.)
- COMPLETE PHYSICAL CHECKUP DATA

EP 3 657 430 A1

# FIG.13

830

## SEARCH RESULT

| | BLOOD SUGAR LEVEL | BLOOD PRESSURE | MEDICAL HISTORY | MEDICATION | AGE | GENDER | DOMICILE |
|---|---|---|---|---|---|---|---|
| 831 ☑ | 80(mg/dℓ) | 110(mmHg) | ①*** ②**** ③**** | ①*** ②*** | 40 | MALE | TOKYO |
| 832 ☐ | 70(mg/dℓ) | 120(mmHg) | ①*** ②**** | ①*** | 51 | FEMALE | KANAGAWA |
| 833 ☑ | **(mg/dℓ) | ***(mmHg) | ①*** | | 38 | MALE | FUKUOKA |

| REGISTER IN TARGET USER LIST | VIEW DETAIL DATA |
|---|---|

836

835

EP 3 657 430 A1

# FIG.14

850

## YOUR HEALTH SCORE

| BLOOD SUGAR LEVEL | BLOOD PRESSURE | ... |

BLOOD SUGAR LEVEL HEALTH SCORE

**34**

☺

**HEALTH ADVICE**
YOU HAVE DEVELOPED TENDENCY OF BLOOD SUGAR LEVEL SPIKE AFTER MEAL SINCE LAST YEAR. DAILY MEAL SHOULD ... YOU HAVE RISK OF XX DISEASE... TO IMPROVE YOUR HEALTH, ...

OO TEA IS RECOMMENDED FOR YOUR BLOOD SUGAR LEVEL

# FIG.15

## INTERNATIONAL SEARCH REPORT

| International application No. |
| --- |
| PCT/JP2018/017506 |

**A. CLASSIFICATION OF SUBJECT MATTER**
Int.Cl. G06Q50/22(2018.01)i, A61B5/00(2006.01)i

According to International Patent Classification (IPC) or to both national classification and IPC

**B. FIELDS SEARCHED**

Minimum documentation searched (classification system followed by classification symbols)
Int.Cl. G06Q10/00-99/00, G16H10/00-80/00, A61B5/00, G06T1/00, G09F19/00

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2007-323528 A (MATSUSHITA ELECTRIC INDUSTRIAL CO., LTD.) 13 December 2007, paragraphs [0029]– | 1-4, 7-8, 17-20 |
| Y | [0049], [0066]–[0067], [0079]–[0086], [0155] (Family: none) | 5-6, 9-16 |
| Y | JP 2015-111438 A (HEALTHGRID INC.) 18 June 2015, paragraphs [0023]–[0026], [0033]–[0035] (Family: none) | 5-6, 16 |

☒ Further documents are listed in the continuation of Box C. ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 09 July 2018 (09.07.2018) | 17 July 2018 (17.07.2018) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2018/017506

C (Continuation).   DOCUMENTS CONSIDERED TO BE RELEVANT

| Category* | Citation of document, with indication, where appropriate, of the relevant passages | Relevant to claim No. |
|---|---|---|
| Y | WO 2011/096240 A1 (NEC CORP.) 11 August 2011, paragraphs [0018]-[0019], [0027]-[0030] & US 2012/0296571 A1, paragraphs [0045]-[0051], [0072]-[0081] & CN 102740773 A | 6 |
| Y | JP 2000-148985 A (HITACHI, LTD.) 30 May 2000, paragraphs [0033]-[0034] (Family: none) | 6 |
| Y | JP 2006-262993 A (NIPPON TELEGRAPH AND TELEPHONE CORP.) 05 October 2006, paragraphs [0014]-[0015] (Family: none) | 9 |
| Y | JP 2004-358231 A (SEIKO INSTRUMENTS INC.) 24 December 2004, paragraphs [0032]-[0036], [0085]-[0099] & US 2005/0159652 A1, paragraphs [0044]-[0048], [0099]-[0113] & EP 1477111 A1 & CA 2467306 A1 | 10-16 |
| Y | JP 2006-258978 A (OMRON HEALTHCARE CO., LTD.) 28 September 2006, paragraphs [0016]-[0017], [0051]-[0055], [0062] (Family: none) | 13-15 |
| A | US 2013/0262155 A1 (HINKAMP, Thomas J.) 03 October 2013, paragraphs [0025]-[0027], [0040], [0108], [0122]-[0126] (Family: none) | 1-20 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2013117941 A